# EUROPEAN PATENT SPECIFICATION

(11) **EP 0 642 338 B1**
(45) Date of publication and mention of the grant of the patent: **22.01.1997**
(21) Application number: 93909326.6
(22) Date of filing: 14.04.1993
(51) Int. Cl.: A61K 31/135

(54) **USE OF (E)-2-(p-FLUOROPHENETHYL)-3-FLUOROALLYLAMINE IN THE TREATEMENT OF ALZHEIMER'S DISEASE**
VERWENDUNG VON (E) - 2 - (P -FLUOROPHENETHYL)- 3 - FLUOROALLYLAMINE ZUR BEHANDLUNG DER ALZHEIMERSCHEN KRANKHEIT
UTILISATION DE (E)-2-(p-FLUOROPHENETHYLE)-3-FLUOROALLYLAMINE DANS LE TRAITEMENT DE LA MALADIE D'ALZHEIMER

(30) Priority: 27.05.1992 US 888671
(43) Date of publication of application: 15.03.1995
(73) Proprietor: MERRELL PHARMACEUTICALS INC., Cincinnati, Ohio 45215-6300 (US)
(72) Inventor: McDONALD, Ian, A., Solana Beach, CA 92075 (US); PALFREYMAN, Michael, G., Cincinnati, OH 45249 (US)
(74) Representative: VOSSIUS & PARTNER
(86) International application number: US9303522
(87) International publication number: WO9324120

(56) References cited:
- EP-A- 0 295 604
- BIOCHEM. PHARMACOL., vol. 43, no. 2, February 1992, pages 307-12
- DEMENTIA, vol. 1, no. 6, 1990, pages 323-348
- EUR. NEUROL., vol. 31, no. 2, 1991, pages 100-107
- J. Neurosci. Res., vol. 31, 1992, pp. 394-400
- J. Neurosci. Res., vol. 30, 1991, pp. 666-672

## Description

### BACKGROUND OF THE INVENTION

The class of compounds known as monoamine oxidase (MAO) inhibitors have long been utilized for the treatment of depression. MAO is an enzyme which plays an important role in the metabolic regulation of naturally occurring monoamines. MAO catalyzes the biodegradation of monoamines through oxidative deamination. Among the physiologically active monoamines which are known substrates for MAO are: (a) the so-called "neurotransmitter" monoamines, such as the catecholamines (e.g. dopamine, epinephrine and norepinephrine) and the indoleamines (e.g. tryptamine and 5-hydroxytryptamine), (b) the so-called "trace" amines (e.g. o-tyramine, phenethylamine, tele-N-methylhistamine) and (c) tyramine.

Biochemical and pharmacological studies indicate that the MAO enzyme exists in two forms known as "MAO Type A" (MAO-A) and "MAO Type B" (MAO-B). The two forms differ in their distribution in body organs, in their substrate specificity and in their sensitivity to inhibitors. In general, MAO-A selectively oxidizes the so-called "neurotransmitter" monoamines (epinephrine, norepinephrine and 5-hydroxytryptamine), while MAO-B selectively oxidizes the "trace" monoamines (o-tyramine, phenethylamine and tele-N-methylhistamine). Both MAO-A and MAO-B oxidize tyramine, tryptamine and dopamine. However, in man, dopamine has been shown to be a preferred substrate for MAO-B. MAO-A and MAO-B also differ in their sensitivity to inhibition, and thus can be selectively inhibited depending upon the chemical structure of the inhibitor and/or the relative concentrations of the inhibitor and the enzyme. It should be observed that the "selectivity" of an MAO inhibitor arises because the inhibitor has a greater affinity for one form of the enzyme over the other. Thus the selectivity or an inhibitor for MAO-A or MAO-B will be dose-dependent, selectivity being lost as the concentration of inhibitor is increased. For example, L-deprenyl is a selective inhibitor of MAO-B in vivo at lower doses but becomes a non-selective inhibitor of both MAO-A and MAO-B as the dose is increased.

There is now evidence that patients with Alzheimer's disease have higher cerebral MAO-B activity than healthy, elderly people. Monoamines are known to play a fundamental role in the cognitive processes linked to memory and learning, and it has been shown that patients with Alzheimer's disease have reduced activity of different neurotransmission systems mediated by monoamines such as dopamine, noradrenaline and 5-hydroxytryptamine. Finally, the MAO-B inhibitor L-deprenyl now appears to be an effective treatment for patients with Alzheimer's disease. [*See* Mangoni et al., *Eur. Neurol.* 31, 100 (1991)].

The compound (E)-2-(p-fluorophenethyl)-3-fluoroallylamine is a known selective inhibitor of MAO-B with activity as an antiparkinsonian agent. Thus in Dementia, 1 (1990) pp 323 - 348 this compound, designated MDL 72974 and deprenyl are disclosed as being MAO-B inhibitors. EP-A-0 295 604 discloses that MDL 72974 is a more potent MAO-B inhibitor than deprenyl.

### SUMMARY OF THE INVENTION

The present invention provides the use of the compound (E)-2-(p-fluorophenethyl)-3-fluoroallylamine or a pharmaceutically acceptable salt thereof for the preparation of a medicament for the treatment of Alzheimer's disease.

### DETAILED DESCRIPTION OF THE INVENTION

The compound (E)-2-(p-fluorophenethyl)-3-fluoroallylamine is generically disclosed in U.S. Patent No. 4,454,158, issued June 12, 1984, as an MAO-B inhibitor. This patent is incorporated herein by reference in its entirety. The compound (E)-2-(p-fluorophenethyl)-3-fluoroallylamine is specifically disclosed in European Patent Application Publication No. 0 295 604, published December 21, 1988.

Pharmaceutically acceptable salts are such organic and inorganic salts of the compound (E)-2-(p-fluorophenethyl)-3-fluoroallylamine which are non-toxic and allow for bioavailability. For example, the following acid addition salts are pharmaceutically acceptable: hydrochloric, hydrobromic, sulfonic, sulfuric, phosphoric, nitric, maleic, fumaric, benzoic, ascorbic, pamoic, succinic, methanesulfonic, acetic, propionic, tartaric, citric, lactic, malic, mandelic, cinnamic, palmitic, itaconic and benzenesulfonic acid.

In general, (E)-2-(p-fluorophenethyl)-3-fluoroallylamine may be prepared by procedures which are well known and appreciated in the art such as the procedures described in U.S. Patent No. 4,454,158, issued June 12, 1984, and European Patent Application Publication No. 0 295 604, published December 21, 1988.

In general, (E)-2-(p-fluorophenethyl)-3-fluoroallylamine may be prepared by procedures wherein a diester of p-fluorophenylethylbutyric acid is difluoromethylated in a known manner by first treating the diester with a strong base to produce the corresponding carbanion and then contacting the carbanion with a suitable halomethylating agent. The strong base must be non-nucleophilic and be of sufficient strength to remove a proton from the methine moiety adjacent to the carboxy group of the starting ester. Suitable bases are known in the art, such as are disclosed in European Patent Application Publication No. 0 295 604, published December 21, 1988.

Following difluoromethylation, it is preferred to selectively remove one of the ester groups by acid hydrolysis. To accomplish selective cleavage it is preferred to have a mixed diester wherein one ester group is easily cleaved (e.g. one ester group bears t-butyl, benzyl, diphenylmethyl or triphenylmethyl) while the other bears a straight chain alkyl (e.g. methyl, ethyl, propyl or n-butyl).

The easily cleaved ester group can be selectively hydrolyzed by treatment with an organic or inorganic acid, either with or without an added solvent, using a temperature range of about 0° to about 25° C and a reaction time of about 1 to 10 hours. Ambient temperature is preferred. The choice of the acid for the hydrolysis is not critical, except that the acid should be chosen so that it can be easily removed after the hydrolysis stage. Trifluoroacetic acid is preferred since its low boiling point permits it to be easily removed from the hydrolysis product. When one ester group bears benzyl, diphenylmethyl, or triphenylmethyl and the other is a straight-chain C₁-C₄ alkyl group, the easily cleaved ester group can also be selectively cleaved by subjecting the mixed diester to catalytic hydrogenolysis using conventional procedures: for example, by treatment under a hydrogen atmosphere in the presence of a catalyst (e.g., Pd/C) at ambient temperature for 1 to 48 hours. As will be apparent to those skilled in the art, the ester groups can be chosen so that both groups can be cleaved simultaneously by acid hydrolysis or catalytic hydrogenolysis.

Following selective hydrolysis, the difluoromethylated monoester is converted to its acrylate ester by treatment with a base. The reaction can be performed using an aqueous or non-aqueous solvent with strong bases such as sodium hydroxide and the like, or with weak bases, such as triethylamine or sodium bicarbonate. With strong bases, care must be exercised to avoid using an excess of base to prevent interaction with the double bond. The choice of the base, the reaction solvent and reaction conditions will be apparent to those skilled in the art. A preferred procedure is to use aqueous sodium hydroxide in THF at ambient temperature. In general, a temperature range of 0° to 25°C and a reaction time of 15 minutes to 2 hours can be used.

The acrylate ester is reduced to yield the allyl alcohol. The reducing agent employed for this transformation can be any reagent which is known in the art to be capable of selectively reducing an ester function or carboxylic acid function to the corresponding carbinol in the presence of a double bond. A preferred reducing agent is diisobutylaluminum hydride (DIBAL-H) in hexane, THF, diethyl ether, dichloromethane, or mixtures thereof. In a preferred procedure, a solution of the acrylate methyl ester in THF is cooled to about 0° to -78°C (preferably -60 to -70°C), the DIBAL-H dissolved in hexane is added, and the temperature of the mixture is allowed to rise to ambient temperature. The reaction time can be about 2 to 24 hours.

The allyl alcohol can be converted to the desired allyl primary amine using procedures known in the art to be useful for replacing an allylic hydroxyl group by an allylic primary amino group. A preferred laboratory method involves the direct formation of an imido derivative, preferably the phthalimide, and subsequent cleavage of the imido group to generate the primary amino group. The imido derivative can be prepared conveniently by treating the allyl alcohol with the appropriate imide (i.e., phthalimide, succinimide, or maleimide) in the presence of a triarylphosphine (e.g., triphenylphosphine) or a trialkylphosphine and diethyl azodicarboxylate in an aprotic organic solvent (e.g., THF or dioxane). The reaction can be performed using a temperature range of about 0° to 70°C and a reaction time of about 1 to 24 hours. Ambient temperature is preferred. The imido derivative can be cleaved, preferably by reaction with hydrazine in an organic solvent, such as an alkanol (e.g., ethanol) at reflux temperature (50° to 100°C) and a reaction time of about 30 minutes to 10 hours. It is preferable to add an acid (e.g., hydrochloric acid) after the hydrazine treatment to convert the product to the acid addition salt. Other reagents can be used to cleave the imido function. For example, the imide can be heated with a strong mineral acid (e.g., hydrochloric or sulfuric acid) or a mixture of hydrochloric acid and acetic acid. Acids such as hydrobromic acid which are reactive towards olefins usually cannot be used. The final products are conveniently purified and isolated as the acid addition salt using conventional purification methods.

The foregoing procedures may be illustrated by the following example.

### EXAMPLE 1

### (E)-(p-Fluorophenethyl)-3-fluoroallylamine HCl

### Step A: Ethyl 2-(tert-butoxycarbonyl)-p-fluorophenylbutyrate

Treat a solution of p-fluorophenylbutyric acid (25g) in tert-butyl acetate (349mL) with perchloric acid (1.77mL) and then stir at ambient temperature for 1.5 hours. Pour the solution into water (350mL) containing NaOH (48g) and isolate the tert-butyl ester by ether extraction to give a pale yellow oil. Prepare a solution of lithium diisopropylamide from diisopropylamine (22.74g) and 1.6M n-butyl lithium (143.7mL) in THF (200mL), cool to -78°C and slowly add a solution of tert-butyl p-fluorophenylbutyrate (26.76g) in THF (100mL). After 1 hour, add a solution of ethyl chloroformate (12.19g) in THF (100mL) and continue stirring at ambient temperature for 24 hours. Then pour the mixture into water, neutralize with dilute aqueous HCl and isolate the product by ether extraction to give an orange oil (32.27g).

### Step B: Ethyl 2-(tert-butoxycarbonyl)-2-(difluoromethyl)-p-fluorophenylbutyrate

To a solution of crude ethyl 2-(tert-butoxycarbonyl)-p-fluorophenylbutyrate (32.14g) in THF (400mL), add sodium tert-butoxide (19.81g). Stir the mixture for 1 hour, then heat to 45°C and add ClCHF₂ gas rapidly for about 15 minutes. Continue stirring for 1 hour under an atmosphere of ClCHF₂ and allow the temperature to fall to ambient. Pour the reaction mixture into water/brine and isolate the crude product by ether extraction to give an orange oil (34.55g).

### Step C: (E)-Ethyl 2-(p-fluorophenethyl)-3-fluoroacrylate

Stir a solution of ethyl 2-(tert-butoxycarbonyl)-2-(difluoromethyl)-p-fluorophenylbutyrate (30.28g) in trifluoroacetic acid (168mL) for 1 hour and then remove the excess trifluoroacetic acid by evaporation. Dissolve the residual oil (25.82g) in THF (230mL) and slowly treat with 2M NaOH (80mL) so that the pH does not rise above 7.02. After completion of addition of the solution, stir the solution for another 15 minutes and then extract the product into ether. Evaporate the ether and filter the residue through a short column of silica using 5% ethyl acetate in light petroleum as the solvent. Evaporate the solvent to give an essentially pure product as a pale orange oil (15.75g).

### Step D: (E)-2-(p-Fluorophenethyl)-3-fluoroallyl alcohol

Cool a solution of (E)-ethyl 2-(p-fluorophenethyl)-3-fluoroacrylate (15.70g) in hexane (350mL) to -10°C and then slowly treat with a solution of diisobutylaluminum hydride in hexane (1M solution, 196mL). Stir at ambient temperature for 90 minutes, then cool to 10°C and treat consecutively with methanol (196mL) and 6M aqueous HCl (245mL). Add water and isolate the product by ether extraction followed by distillation of the solvents to gave almost pure alcohol (11.36g).

### Step E: (E)-1-Fluoro-2-(p-fluorophenethyl)-3-phthalimidopropene

Cool a solution of (E)-2-(p-fluorophenethyl)-3-fluoroallyl alcohol (11.36g), phthalimide (8.43g) and triphenylphosphine (15.3g) in THF (400mL) to 0°C, and treat slowly with a solution of diethyl azodicarboxylate (9.99g) in THF (50mL). Continue stirring at ambient temperature overnight, then evaporate the solution to leave an orange paste (30g). Separate the pure product by using chromatography on silica (20% ethyl acetate in petroleum ether as eluant) to give a pale yellow solid (13.9g).

### Step F: (E)-(p-Fluorophenethyl)-3-fluoroallylamine HCl

Reflux a mixture of (E)-1-fluoro-2-(p-fluorophenethyl)-3-phthalimidopropene (0.26g) and hydrazine hydrate (80mg) in ethanol (5mL) for 2.5 hours. Add 6N HCl (1.2mL) and evaporate the mixture to dryness. Dissolve the residue in NaOH (10mL) and isolate the crude amine by ether extraction. Dissolve in THF (10mL) and treat with di-tert-butyl dicarbonate (194mg). Reflux the solution for 2 hours and then isolate the crude N-Boc derivative by ether extraction. Purify by silica chromatography (25% ethyl acetate in petroleum ether) to give pure material (180mg) as an almost colorless oil. Dissolve in HCl-saturated ether (12mL) and allow to stand overnight. Filter to give the title product (30mg) as colorless plates (m.p. 131°C).

The present invention provides a method of treatment for Alzheimer's disease in a patient in need thereof comprising administering to said patient a therapeutically effective amount of (E)-2-(p-fluorophenethyl)-3-fluoroallylamine or a pharmaceutically acceptable salt thereof. As used herein, the term "patient" refers to a warm-blooded animal, such as a human, which is afflicted with Alzheimer's disease. The term "patient in need thereof" refers to a patient in need of treatment for Alzheimer's disease.

Alzheimer's disease, also known as Senile Dementia of the Alzheimer's Type (SDAT), is a form of presenile degenerative dementia due to atrophy of frontal and occipital lobes of the brain. Alzheimer's disease involves a progressive loss of memory, deterioration of intellectual functions, apathy, speech and gait disturbances and disorientation. The course of the disease may take a few months to four to five years to progress from the early stages to a complete loss of intellectual function. An attending diagnostician, as one skilled in the art, can identify those patients who are afflicted with Alzheimer's disease on the basis of standard diagnostic procedures and tests.

In effecting treatment according to the present invention, Alzheimer's disease in a patient will be controlled so that the progressive loss of memory, deterioration of intellectual functions, apathy, speech and gait disturbances and disorientation will be slowed, interrupted, arrested or stopped. Treatment will not necessarily result in total elimination of the disease or in regression of the disease to a normal cognitive state.

A therapeutically effective amount of (E)-2-(p-fluorophenethyl)-3-fluoroallylamine, or a pharmaceutically acceptable salt thereof, is an amount which is effective, upon single or multiple dose administration to the patient, in controlling the Alzheimer's disease so that the progressive loss of memory, deterioration of intellectual functions, apathy, speech and gait disturbances and disorientation will be slowed, interrupted, arrested or stopped.

A therapeutically effective amount of (E)-2-(p-fluorophenethyl)-3-fluoroallylamine, or a pharmaceutically acceptable salt thereof, can be readily determined by the attending diagnostician, as one skilled in the art, by the use of known techniques and by observing results obtained under analogous circumstances. In determining the therapeutically effective amount or dose, a number of factors are considered by the attending diagnostician, including, but not limited to: the patient's size, age and general health; the severity of the disease; the response of the individual patient; the mode of administration; the bioavailability characteristics of the preparation administered; the dose regimen selected; the use of concomitant medication; and other relevant circumstances.

A therapeutically effective amount of (E)-2-(p-fluorophenethyl)-3-fluoroallylamine, or a pharmaceutically acceptable salt thereof, will vary from about 0.001 mg/Kg/day to about 1.0 mg/Kg/day. Preferred amounts are expected to vary from about 0.01 mg/Kg/day to about 0.25 mg/Kg/day.

In effecting treatment of a patient afflicted with Alzheimer's disease, the compound (E)-2-(p-fluorophenethyl)-3-fluoroallylamine, or a pharmaceutically acceptable salt thereof, can be administered in any form or mode which makes the compound bioavailable in effective amounts, including oral and parenteral routes. For example, the compound can be administered orally, subcutaneously, intramuscularly, intravenously, transdermally, intranasally, rectally, and the like. Oral administration is generally preferred. Transdermal administration is also preferred. One skilled in the art of preparing formulations can readily select the proper form and mode of administration depending upon the particular characteristics of the compound selected, the stage of the disease, and other relevant circumstances.

The compounds can be administered alone or in the form of a pharmaceutical composition in combination with pharmaceutically acceptable carriers or excipients, the proportion and nature of which are determined by the solubility and chemical properties of the compound selected, the chosen route of administration, and standard pharmaceutical practice. The compounds used in accordance with the invention, while effective themselves, may be formulated and administered in the form of their pharmaceutically acceptable acid addition salts for purposes of stability, convenience of crystallization, increased solubility and the like.

The pharmaceutical compositions are prepared in a manner well known in the pharmaceutical art. The carrier or excipient may be a solid, semi-solid, or liquid material which can serve as a vehicle or medium for the active ingredient. Suitable carriers or excipients are well known in the art. The pharmaceutical composition may be adapted for oral or parenteral use and may be administered to the patient in the form of tablets, capsules, suppositories, solution, suspensions, transdermal patch or the like.

The compounds used in accordance with the present invention may be administered orally, for example, with an inert diluent or with an edible carrier. They may be enclosed in gelatin capsules or compressed into tablets. For the purpose of oral therapeutic administration, the compounds may be incorporated with excipients and used in the form of tablets, troches, capsules, elixirs, suspensions, syrups, wafers, chewing gums and the like. These preparations should contain at least 0.1% of the compound of the invention, the active ingredient, but may be varied depending upon the particular form and may conveniently be between 0.5% to about 20% of the weight of the unit. The amount of the compound present in compositions is such that a suitable dosage will be obtained. Preferred compositions and preparations used according to the present invention are prepared so that an oral dosage unit form contains between 0.05-25 milligrams of a compound of the invention.

The tablets, pills, capsules, troches and the like may also contain one or more of the following adjuvants: binders such as microcrystalline cellulose, gum tragacanth or gelatin; excipients such as starch or lactose, disintegrating agents such as alginic acid, Primogel™, corn starch and the like; lubricants such as magnesium stearate or Sterotex™; glidants such as colloidal silicon dioxide; and sweetening agents such as sucrose or saccharin may be added or a flavoring agent such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the present compounds, sucrose as a sweetening agent and certain preservatives, dyes and colorings and flavors. Materials used in preparing these various compositions should be pharmaceutically pure and non-toxic in the amounts used.

For the purpose of parenteral therapeutic administration, such as intramuscular, intravenous, and subcutaneous, the compounds used in accordance with the present invention may be incorporated into a solution or suspension. These preparations should contain at least 0.01% of a compound of the invention, but may be varied to be between 0.01 and about 50% of the weight thereof. The amount of the inventive compound present in such compositions is such that a suitable dosage will be obtained. Preferred compositions and preparations according to the present invention are prepared so that a parenteral dosage unit contains between 0.01 to 10 milligrams of the compound of the invention.

The solutions or suspensions may also include one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

The compounds used in accordance with this invention can also be administered topically. This can be accomplished by simply preparing a solution of the compound to be administered, preferably using a solvent known to promote transdermal absorption such as ethanol or dimethyl sulfoxide (DMSO) with or without other excipients. Preferably topical administration will be accomplished using a patch either of the reservoir and porous membrane type or of a solid matrix variety.

Some suitable transdermal devices are described in U.S. Pat. Nos. 3,742,951, 3,797,494, 3,996,934, and 4,031,894. These devices generally contain a backing member which defines one of its face surfaces, an active agent permeable adhesive layer defining the other face surface and at least one reservoir containing the active agent interposed between the face surfaces. Alternatively, the active agent may be contained in a plurality of microcapsules distributed throughout the permeable adhesive layer. In either case, the active agent is delivered continuously from the reservoir or microcapsules through a membrane into the active agent permeable adhesive layer, which is in contact with the skin or mucosa of the recipient. If the active agent is absorbed through the skin, a controlled and predetermined flow of the active agent is administered to the recipient. In the case of microcapsules, the encapsulating agent may also function as the membrane.

In another device for transdermally administering the compounds in accordance with the present invention, the pharmaceutically active compound is contained in a matrix from which it is delivered in the desired gradual, constant and controlled rate. The matrix is permeable to the release of the compound through diffusion or microporous flow. The release is rate controlling. Such a system, which requires no membrane is described in U.S. Pat. No. 3,921,636. At least two types of release are possible in these systems. Release by diffusion occurs when the matrix is non-porous. The pharmaceutically effective compound dissolves in and diffuses through the matrix itself. Release by microporous flow occurs when the pharmaceutically effective compound is transported through a liquid phase in the pores of the matrix.

## Claims

1. Use of the compound (E)-2-(p-fluorophenethyl)-3-fluoroallylamine or a pharmaceutically acceptable salt thereof for the preparation of a medicament for the treatment of Alzheimer's disease.

2. Use of the compound according to claim 1 wherein the salt is selected from the acid addition salts consisting of hydrochloric, hydrobromic, sulfonic, sulfuric, phosphoric, nitric, maleic, fumaric, benzoic, ascorbic, pamoic, succinic, methanesulfonic, acetic, propionic, tartaric, citric, lactic, malic, mandelic, cinnamic, palmitic, itaconic and benzenesulfonic acid.

3. Use according to claim 1 wherein the compound is (E)-2-(p-fluorophenethyl)-3-fluoroallylamine hydrochloric acid.

## Patentansprüche

1. Verwendung der Verbindung (E)-2-(p-Fluorphenethyl)-3-fluorallylamin oder eines pharmazeutisch verträglichen Salzes davon zur Herstellung eines Arzneimittels zur Behandlung der Alzheimer'schen Krankheit.

2. Verwendung der Verbindung nach Anspruch 1, wobei das Salz aus Säureadditionssalzen mit Chlorwasserstoffsäure, Bromwasserstoffsäure, Sulfonsäure, Schwefelsäure, Phosphorsäure, Salpetersäure, Maleinsäure, Fumarsäure, Benzoesäure, Ascorbinsäure, Pamoasaure, Bernsteinsäure, Methansulfonsäure, Essigsaure, Propionsäure, Weinsäure, Zitronensäure, Milchsäure, Äpfelsäure, Mandelsäure, Zimtsäure, Palmitinsäure, Itaconsäure und Benzolsulfonsäure ausgewählt ist.

3. Verwendung nach Anspruch 1, wobei die Verbindung das Chlorwasserstoffsäure-Salz von (E)-2-(p-Fluorphenethyl)-3-fluorallylamin ist.

## Revendications

1. Utilisation du composé (E)-2-(p-fluorophénéthyl)-3-fluoroallylamine ou d'un de ses sels pharmaceutiquement acceptables pour la préparation d'un médicament destiné au traitement de la maladie d'Alzheimer.

2. Utilisation du composé selon la revendication 1, dans laquelle le sel est choisi parmi les d'addition d'acides constitués par l'acide chlorhydrique, l'acide bromhydrique, l'acide sulfonique, l'acide sulfurique, l'acide phosphorique, l'acide nitrique, l'acide maléique, l'acide fumarique, l'acide benzoïque, l'acide ascorbique, l'acide pamoïque, l'acide succinique, l'acide méthanesulfonique, l'acide acétique, l'acide propionique, l'acide tartrique, l'acide citrique, l'acide lactique, l'acide malique, l'acide mandélique, l'acide cinnamique, l'acide palmitique, l'acide itaconique et l'acide benzènesulfonique.

3. Utilisation selon la revendication 1, dans laquelle le composé est le chlorhydrate de l'(E)-2-(p-fluorophénéthyl)-3-fluoroallylamine.
